(19) **Europäisches Patentamt**
**European Patent Office**
**Office européen des brevets**

(11) **EP 1 713 378 B1**

(12) **EUROPÄISCHE PATENTSCHRIFT**

(45) Veröffentlichungstag und Bekanntmachung des Hinweises auf die Patenterteilung:
**08.05.2013 Patentblatt 2013/19**

(21) Anmeldenummer: **05726333.7**

(22) Anmeldetag: **04.02.2005**

(51) Int Cl.:
*A61B 3/10* *(2006.01)*      *A61B 3/12* *(2006.01)*
*G01B 9/02* *(2006.01)*      *G01B 11/14* *(2006.01)*

(86) Internationale Anmeldenummer:
**PCT/EP2005/001164**

(87) Internationale Veröffentlichungsnummer:
**WO 2005/074789 (18.08.2005 Gazette 2005/33)**

(54) **KURZKOHÄRENZ-INTERFEROMETRISCHE LÄNGENMESSUNG AM AUGE**

SHORT-COHERENCE INTERFEROMETRIC MEASUREMENT OF LENGTH ON THE EYE

MESURE DE LONGUEUR INTERFEROMETRIQUE A COHERENCE COURTE SUR L'OEIL

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **06.02.2004  AT 1792004**
**27.04.2004  AT 7192004**

(43) Veröffentlichungstag der Anmeldung:
**25.10.2006  Patentblatt 2006/43**

(73) Patentinhaber: **Carl Zeiss Meditec AG**
**07745 Jena (DE)**

(72) Erfinder: **FERCHER, Adolf, Friedrich**
**A-1230 Wien (AT)**

(56) Entgegenhaltungen:
**WO-A-02/04884      WO-A-92/19930**
**WO-A-03/086180      DE-A1- 19 624 167**

**Beschreibung**

Die vorliegende Anmeldung betrifft ein Messverfahren der diagnostischen Ophthalmologie, insbesondere Messanordnungen für die Messung von Teilstreckenlängen am Auge im Zusammenhang mit der Katarakt-Chirurgie und der refraktiven Augenchirurgie.

Stand der Technik: Bei der Katarakt-Chirurgie und der refraktiven Augenchirurgie wird durch geeignete Wahl der Brechkraft der zu implantierenden Intraokularlinse eine bestimmte Brechkraft des Auges erhalten oder erzielt. Hierzu ist die Messung des refraktiven Ausgangszustands des Auges und, gegebenenfalls, nach erfolgter Operation, auch die Messung des refraktiven Endzustands erforderlich. Hierzu finden Keratometer zur Messung der Hornhautkrümmung Verwendung und akustische oder optische Längenmessverfahren zur Bestimmung der axialen Teilstrecken des Auges.

Die Bestimmung der axialen Augenlänge erfolgt heute bereits sehr häufig mittels optischer Kurzkohärenz-Interferometrie, die gegenüber der bisher dominierenden Ultraschall-Methode die Vorteile der berührungsfreien und hochpräzisen Arbeitsweise hat. Bei der Kurzkohärenz-Interferometrie treten Interferenzen nur dann auf, wenn sich Objekt und Referenzspiegel bis auf die Kohärenzlänge $l_C$ im gleichen optischen Abstand vom Strahlteiler befinden, oder, anders ausgedrückt, die betreffende Objektstruktur befindet sich "im Kohärenzfenster". Zur Messung der Distanzen von Objektstrukturen werden diese - bei der üblichen time-domain-Methode - durch kontrolliertes Verschieben eines Spiegels im Referenz- oder Messstrahl zeitlich hintereinander in das Kohärenzfenster gebracht. Die Messgenauigkeit ist daher durch die Kohärenzlänge $l_C$ des Messlichtbündels gegeben, welche neben der Wellenlänge $\lambda$ hauptsächlich von dessen spektraler Breite $\Delta\lambda$ abhängt (genau genommen spielt auch die Form des Spektrums eine Rolle):

$$l_C \sim \lambda^2/\Delta\lambda. \qquad (1)$$

Das Kohärenzfenster hat in der ophthalmologischen Kurzkohärenz-Interferometrie meist eine Länge (= $l_C$) von einigen Mikrometern.

Im Gegensatz zu der klassischen optischen Kurzkohärenz-Interferometrie, bei der der Referenzspiegel des Interferometers die gesamte zu messende Wegstrecke abfährt oder "scannt", hat man für die ophthalmologische Augenlängenmessung spezielle Verfahren entwickelt. Diese erlauben es, trotz Distanzen am Auge bis zu etwa 30 Millimeter, diese auch an instabilen, weil lebenden Objekten, zu messen. Einer der Auswege zur Lösung dieses Problems bei der kurzkohärenz-interferometrischen Messung des Abstands von in der Tiefe separierten instabilen Strukturen ist die Verwendung des sogenannten Dual-Beam Verfahrens. Dieses Verfahren wird in der Offenlegungsschrift DE 3201801A1 beschrieben. Dabei wird die Cornea und die in der Tiefe separierte andere Augenstruktur, beispielsweise der Fundus, von einem Doppel-Messstrahl beleuchtet. Dieser duale Messstrahl wird von zwei Ausgangsstrahlen eines Michelson-Interferometers unterschiedlicher Weglängen gebildet. Mit Hilfe einer diffraktiven Linse wird dieser Messstrahl gleichzeitig auf Cornea und auf Fundus fokussiert. Das Michelson-Interferometer wird auf die Distanz Comea/Fundus abgestimmt. Dazu genügt eine Interferometerspiegel-Scan-Strecke von wenigen Millimetern. Dieser Abgleich wird mittels der hierbei auftretenden Interferenzen von Kurzkohärenz-Licht festgestellt. Da hiefür nur die Distanz Cornea/Fundus ausschlaggebend ist, werden interferometrische Stabilitätsanforderungen in idealer Weise erfüllt; die Messung bleibt von Bewegungen des Auges unbeeinträchtigt.

Auch bei den in der Patentanmeldung WO 01/38820A1 beschriebenen Verfahren werden die zwei in der Tiefe distanzierten Objektbereiche mit einem Doppel-Messstrahl beleuchtet. Hier wird aus dem das Messobjekt beleuchtenden Messstrahl vor dem Messobjekt zunächst ein weiterer Messstrahl mittels eines Strahlteilers ausgespiegelt und nach Durchlaufen eines Umwegs, in dem auch eine zusätzliche brechende Optik zur Fokussierung angeordnet sein kann, wieder in den ursprünglichen Messstrahl eingespiegelt. Dieses Verfahren reduziert die Interferometerspiegel-Scan-Strecke auf einen kleineren Wert als die zu messende Wegstrecke. Bei entsprechend schnellem Scannen kann dieses Verfahren die interferometrischen Stabilitätsanforderungen ebenfalls erfüllen.

Die angeführten Verfahren haben jedoch den Nachteil, daß das Messlicht gleichzeitig zwei oder mehr distanzierte Objektbereiche beleuchtet. Das jeweils nicht zur Messung benutzte Licht erzeugt einen störenden Untergrund und Rauschen. Ferner ist dadurch ein Fokussieren des Messlichts auf die jeweilige Messstelle schwierig zu realisieren; insbesondere bei mehreren distanzierten Objektbereichen wird diese Problematik gravierend. Die moderne ophthalmologische Längenmessung am Auge erfordert jedoch mehr als eine Strecke zu messen, nämlich zusätzlich zur Augenlänge noch Distanzen wie die Vorderkammertiefe und die Cornea-und Augenlinsendicke.

In der DE 196 24 167 A1 ist eine Lösung nach dem Oberbegriff von Anspruch 1 offenbart.

Es ist daher die technische Aufgabe der Erfindung, Kurzkohärenz-Interferometer zur Messung der Teilstrecken des Auges anzugeben, die den Messstrahl auf die jeweiligen Kohärenzfenster fokussieren und weiters die erforderlichen Interferometerspiegel-Scan-Strecken auf kleinere Strecken als die zu messende Distanz reduzieren.

Dies wird erfindungsgemäß durch ein Kurzkohärenz-Interferometer gelöst, in dem ein 90-Grad-Spiegel und eine Fokussieroptik gemeinsam eine periodische Hin-und-her Bewegung so ausführen, dass der von der Fokussieroptik erzeugte und mittels einer Relais-Optik auf das Auge abgebildete Messstrahlfokus synchron mit dem Kohärenzfenster von der Cornea entlang der optischen Achse des Auges bis zur Fovea centralis bewegt wird und weiters mittels mehrerer Reflektoren unterschiedliche Weglängen in den Mess- und Referenzstrahlengängen erzeugt werden.

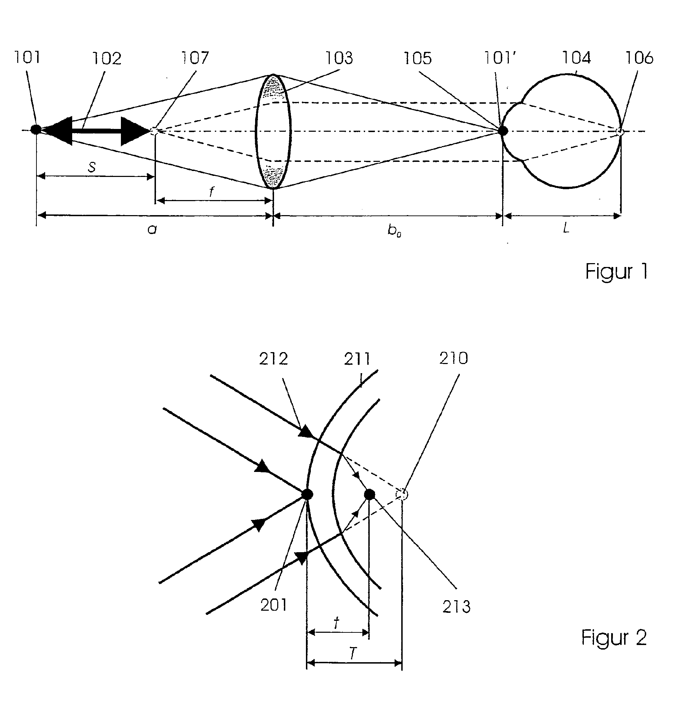

Die Figur 1 erläutert zunächst die geometrische Optik des Tiefenscans am Auge: 101 ist der bewegte Fokus des Messstrahls eines Kurzkohärenz-Interferometers, der sich entlang der durch den Doppelpfeil 102 angedeuteten Strecke der Länge S bewegt. Der Fokus 101 wird mittels einer Relais-Optik 103 auf das Auge 104 abgebildet. Die kurzkohärenz-interferometrische Bedingung ist hierbei, dass für eine Bewegung des Fokus 101 um die optische Länge $S = L$ des Auges (beim Gullstrand-Auge ist $L$ etwa gleich 33 mm), das Bild 101' des Fokus 101 die gesamte Länge des Auges 104 vom Corneascheitel 105 bis zur Fovea centralis 106 abtasten soll. Es soll also der Punkt 107 am Ende dieser Bewegung in den Punkt 106 abgebildet werden. Dies erreicht man für eine Optik der Brennweite $f$, wenn sich der Corneascheitel im Abstand

$$b_0 = \frac{f(f+L)}{L} \tag{2}$$

von der Relais-Optik 103 befindet. Benutzt man beispielsweise eine Optik der Brennweite $f = 33$ mm, so ist für das Gullstrand-Auge $b = 66$ mm.

Für ein gutes kurzkohärenz-interferometrisches Signal muß weiters der Messstrahl innerhalb des Kohärenzfensters auf das Objekt fokussiert werden. Die in der Figur 1 beschriebene Anordnung gewährleistet zwar, dass, wenn das Kohärenzfenster an der Cornea mit dem Fokus 101' zusammenfällt, es dann auch an der Fovea centralis mit dem Fokus des Messstrahls im Punkt 106 zusammenfällt, jedoch ist das auf der dazwischenliegenden Strecke nicht vollständig gewährleistet. Das sieht man, wenn man den Verlauf der Fokusposition vom Corneascheitel 201 (Figur 2) in das Innere des Auges verfolgt. Wegen der Lichtbrechung an der Cornea 211 verschiebt sich der Fokus eines auf einen Punkt 210 hinter der Cornea 211 fokussierten Lichtbündels 212 um eine geringere Strecke ($t$) als das Lichtbündel selbst ($T$) und damit das Kohärenzfenster. Der neue Fokus liegt nicht im Punkt 210 sondern bei 213, siehe Figur 2. Außerdem liegt das Kohärenzfenster nicht bei 210, sondern in der optischen Distanz $n_G \cdot T$, mit $n_G$ = Gruppenindex, vom Scheitel entfernt.

Dieses Auseinanderlaufen von Kohärenzfenster und Messbündelfokus ist besonders bei Messungen im vorderen Augenabschnitt von Bedeutung, weil dort Strukturen liegen (Rückfläche der Cornea, Vorder- und Rückfläche der Augenlinse), die mit hoher Präzision gemessen werden sollten. Man kann dieses Auseinanderlaufen für den vorderen Augenabschnitt dadurch minimieren, dass man die Anfangskoinzidenz von Fokus 210 und Kohärenzfenster nicht - wie üblich - für Punkte außerhalb des Auges, also beispielsweise für den Corneascheitel, sicherstellt, sondern für einen Punkt etwa in der Mitte der Vorderkammer, beispielsweise 3 mm hinter dem Scheitel. Dies wird durch entsprechende Positionierung des Reflektors für das Kohärenzfenster der Cornea erreicht. Hierzu wird die Interferometer-Armlänge mittels des Justiertisches 358, 558 oder 758 eingestellt.

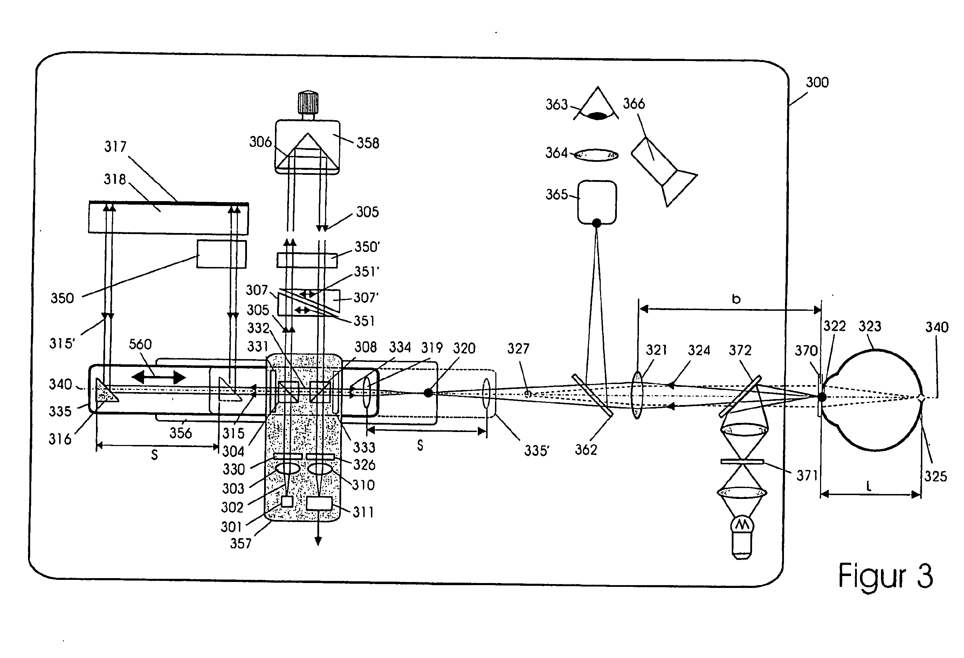

Eine erste erfindungsmäßige Anordnung ist in der Figur 3 dargestellt. Dort emittiert eine Kurzkohärenz-Lichtquelle 301, beispielsweise eine Superlumineszenzdiode, einen zeitlich teilkohärenten und räumlich möglichst vollständig kohärenten Lichtstrahl 302, der mittels einer Optik 303 den Strahlteiler 304 beleuchtet. Der Strahlteiler 304 teilt den Lichtstrahl 302 in Messstrahl 315 und Referenzstrahl 305. Der Referenzstrahl 305 wird von dem Referenzspiegel 306 in Richtung Strahlteiler 308 reflektiert. Er durchsetzt dabei 2 mal die zwei Dispersions-Kompensationsprismen 307 und 307'. Nach Durchlaufen des Strahlteilers 308 trifft der Referenzstrahl 3 05 auf die Optik 310 und wird von dieser auf den Photodetektor 311 fokussiert. Der vom Strahlteiler 304 (in der Zeichnung nach links) reflektierte Messstrahl 315 wird von dem 90-Grad-Spiegel 316 um 90° aus seiner ursprünglichen Richtung reflektiert und trifft auf den Reflektorspiegel 317, der hier als verspiegelte Rückfläche einer Planplatte 318 ausgebildet ist. Es versteht sich von selbst, dass hier auch andere Spiegel verwendet werden können, beispielsweise Oberflächenspiegel mit der Reflektorseite dem eintreffenden Strahl zugewandt. Der reflektierte Messstrahl 315' wird von dem 90-Grad-Spiegel 316 wiederum auf den Strahlteiler 304 gerichtet, durchläuft diesen sowohl als auch den Strahlteiler 308 geradlinig und wird von der Fokussieroptik 319 - als Messstrahl 334 - in den Fokus 320 fokussiert. Der Fokus 320 befindet sich - wie beispielsweise in der Figur 3 gezeichnet - in der doppelten gegenstandseitigen Brennweite der Relais-Optik 321. Die Relais-Optik 321 bildet den Fokus 320 auf das Auge 323 ab. Der Fokus 320 wird - wie beispielsweise in der Figur 3 gezeichnet - in der doppelten bildseitigen Brennweite der Relais-Optik 321 in den Punkt 322 abgebildet. Der Punkt 322 befindet sich auf der Cornea eines Auges 323, dessen Länge L gemessen wird.

Die Strahlteiler 304 und 308 können zur Vermeidung von störenden Reflexionen und zur Optimierung der Strahlintensitäten als polarisierende Strahlteiler ausgebildet werden. Dann kann man durch Drehen eines Linearpolarisators 330 das Teilungsverhältnis Referenzstrahlintensität zu Messstrahlintensität so optimieren, dass man am Photodetektor 311 optimales Signal-zu-Rausch-Verhältnis erhält. Ferner kann man nach dem Strahlteiler 304 im Messstrahl 315 eine λ/4-Platte 331 unter 45° zur Polarisationsrichtung anordnen. Dann wird der Reflektor 317 von einem zirkular polarisiertem Lichtbündel 315 beleuchtet, welches nach Reflexion als Lichtbündel 315' gegenläufig zirkular polarisiert ist und nach einem weiteren Durchsetzen der λ/4-Platte 331 wieder linear polarisiert wird und zwar orthogonal zur ursprünglichen Polarisationsrichtung. Dieser Strahl 332 durchsetzt daher die polarisierenden Strahlteiler 304 und 308 ohne Reflexionsverluste und trifft auf eine weitere λ/4-Platte 333, unter 45° angeordnet. Hier wird der Messstrahl 334 wiederum zirkular polarisiert und von der Fokussieroptik 319 in den Fokus 320 und weiter von der Relais-Optik 321 in den Fokus 322 fokussiert. Der Corneascheitel reflektiert das Lichtbündel 324, nunmehr entgegengesetzt zirkular polarisiert. Dieses zurück laufende Messlichtbündel 324 wird von der λ/4-Platte 333 wiederum linear, orthogonal zu der Polarisationsrichtung des Bündels 332, polarisiert und vom polarisierenden Strahlteiler 308 daher zu 100 % in Richtung Optik 310 reflektiert und von dieser am Photodetektor 311 fokussiert. Auf diese Weise werden im Messstrahl Leistungsverluste weitgehend vermieden.

Der linear polarisierte Referenzstrahl 305 durchsetzt nach der Reflexion am Referenz-Reflexionsprisma 306 die Dispersions-Kompensatoren 307 und 307' und den Strahlteiler 308 und wird ebenfalls von der Optik 310 am Photodetektor 311 fokussiert. Mit dem Linearpolarisator 326 kann die Größe der aus Mess- und Referenzstrahl miteinander interferierenden Komponenten eingestellt werden. 326 wird so orientiert, dass am Photodetektor 311 optimales Signal-zu-Rausch-Verhältnis auftritt.

Das beschriebene Kurzkohärenz-Interferometer ist auf einer Platte 300 aufgebaut. 90-Grad-Spiegel 316 und Fokussieroptik 319 befinden sich auf einer in Richtung der strichpunktiert gezeichneten Interferometerachse 340 um die Strecke S beweglichen Scannerplatte 335 (33 5'). Die Scannerplatte 335 kann der bewegte Schlitten eines Voice Coil Scanners - beispielsweise der Firma Physik Instrumente - oder eines Ultraschall-Scanningtisches oder einer anderen entsprechenden Vorrichtung sein, dessen Fußplatte 356 auf der Grundplatte 300 befestigt ist. Hingegen befinden sich die Komponenten 301, 302, 303, 304, 331, 308, 333, 326, 310, und 311 auf einer fest mit der Grundplatte 300 verbundenen Trägerplatte 357, die die Scannerplatte 335 überbrückt.

Das Reflexionsprisma 306 ist auf einem Verschiebetisch 358 montiert. Seine Position kann so abgestimmt werden, dass sich das Kohärenzfenster bei der in der Figur 3 durch zusammenhängende Linien dargestellten Konfiguration der Scannerplatte 335 des Interferometers am Corneascheitel des Auges 323 befindet. Bewegt sich nun die Scannerplatte 335 - auf dieser sind der 90-Grad-Spiegel 316 und die Fokussieroptik 319 montiert - in Richtung der strichpunktiert gezeichneten Interferometerachse 340 um die Strecke S, dann verkürzt sich der Messstrahlengang in der Zeichnung links vom Strahlteiler 304 um 2 mal S. Die Folge ist, dass sich das Kohärenzfenster um die Strecke S vom Corneascheitel in das Auge verschiebt. Um die Augenlänge zu messen, kann man nun das Kohärenzfenster um die gesamte optische Augenlänge L nach rechts verschieben und diese Strecke anhand der dann auftretenden Kurzkohärenz-Interferenzen des vom Fundus 325 reflektierten Lichts mit dem Referenzlicht abgrenzen. Alternativ kann die Position des Reflexionsprismas 306, wie oben ausgeführt, mit Hilfe des Verschiebetisches 358 so abgestimmt werden, dass der Fokus für einen Punkt etwa in der Mitte der Vorderkammer des Auges in der Mitte des Kohärenzfensters liegt. Eine solche Justierung ist besonders dann von Interesse, wenn es um präzise Messungen der Vorderkammergeometrie geht.

Die Genauigkeit kurzkohärenz-interferometrischer Messungen wird durch Dispersion in den Interferometerarmen beeinträchtigt. Zur Erzielung der maximal möglichen Genauigkeit, die etwa von der Größe der Kohärenzlänge $l_C$ ist, muß die Dispersion in den beiden Interferometerarmen möglichst gleich groß sein - man spricht dann von "Dispersionskompensation". Die durch die Interferometerbauteile bedingte Dispersion kann durch entsprechend gewählte Dikken der Spiegelplatte 318 oder durch zusätzliche Planplatten (350, 350') erreicht werden. Die objektabhängige Dispersion kann durch gegenseitiges Verschieben (Doppelpfeile 351 und 351') zweier Keilplatten (307, 307') im Referenzarm kompensiert werden.

Zur Beobachtung der Position des Probandenauges 323 relativ zum Messstrahl kann im Messstrahl ein teildurchlässiger Spiegel 362 angeordnet werden. Diese Beobachtung kann direkt (363), mit Hilfe eines Okulars 364 oder mit Hilfe einer Fernsehkamera 365 erfolgen. Hierzu kann es sinnvoll sein, das Probandenauge 323 zusätzlich mit einer inkohärenten Lichtquelle 3 66 zu beleuchten. Zur präzisen Positionierung des Probandenauges auf der Interferometerachse 340 kann auch das Bild 370 einer Strichplatte 371 benutzt werden, welches über einen teildurchlässigen Spiegel 372 auf die Probanden-Cornea gespiegelt wird.

Die soweit beschriebenen Verfahren haben den Nachteil, dass die Scannerplatte 335 um die gesamte Augenlänge L verschoben werden muss, was zeitaufwendig ist.

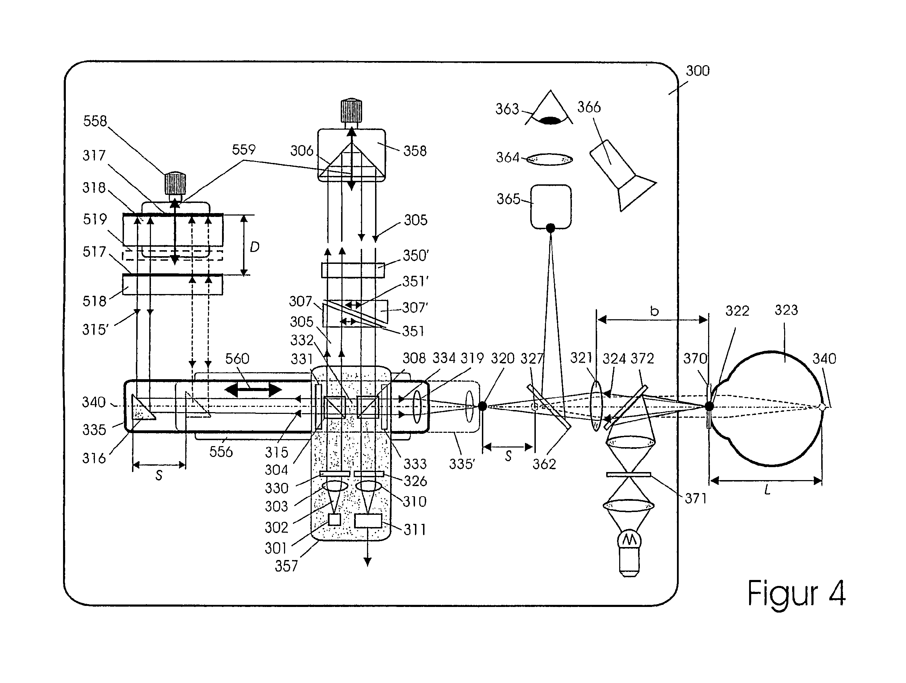

In der vorliegenden Erfindung wird nun vor dem Reflektor 317 ein weiterer Reflektor 517 angeordnet, wie in der Figur 4 dargestellt. Dieser Planspiegel befindet sich von dem Reflektor 317 in einem bekannten Abstand D. An diesem Spiegel wird bereits ein Teil des Messlichtstrahls 315 reflektiert. Dieser Teil des Messlichtstrahls 315' hat gegenüber dem am Reflektor 317 reflektierten Messlicht bereits ein um die optische Länge D am Auge nach rechts versetztes Kohärenzfenster. Hat man das Interferometer so justiert, dass das vom ("Corneaassoziierten") Reflektor 317 reflektierte Messlicht nach Reflexion an der Cornea Kurzkohärenz-Interferenzen mit dem Referenzlicht erzeugt, dann genügt bereits eine Verschiebung der Scannerplatte 335 um die Strecke $S = L - D$ um Kurzkohärenz-Interferenzen des vom ("Fundus-assoziierten") Reflektor 517 auf den Augenfundus gerichteten und dort reflektierten Lichtbündels mit dem Referenzlicht auftreten zu lassen. Aus der so kurzkohärenz-interferometrisch gemessenen Strecke $L - D$ und der bekannten Strecke $D$ erhält man die Augenlänge $L$. Der Abstand des Corneascheitels von der Relais-Optik 321 muß nun

$$b = f + \frac{f^2}{L-D} \qquad (3)$$

betragen.

Die im Zusammenhang mit der Anordnung nach Figur 3 beschriebenen Modifizierungen zur Vermeidung von störenden Reflexionen, zur Dispersionskompensation und zur Beobachtung der Position des Probandenauges lassen sich auch in dem Strahlengang nach Figur 4 sinngemäß realisieren. Man kann ferner die Position des Reflektors 317 und das Dachkantprisma 306 mit Hilfe der Verschiebetische 558 und 358 durch axiales Verschieben (Doppelpfeile 559) so auf die aktuellen Teilstreckenlängen des Auges abstimmen, dass der erforderliche mechanische Scanvorgang durch die Scannerplatte 335 des Kurzkohärenz-Interferometers auf wenige Millimeter reduziert wird. Dadurch wird der Messvorgang am Auge nicht nur erheblich beschleunigt, sondern auch der Einsatz billigerer Scanner ermöglicht.

Weiters können neben der Augenlänge auch die verschiedenen intraokulären Teilstrecken gemessen werden. Hierzu kann man weitere Reflektoren anordnen, die in der Tiefe gestaffelt sind; beispielsweise einen Reflektor 519 so, daß dessen Position etwa der Position der Linsenvorderfläche entspricht. Dann kann man anhand der entsprechenden Kurzkohärenz-Interferenzen auch den Abstand Cornea-Augenlinse messen.

Die Trägerplatten der Reflektoren (318, 518, 519) können in ihrer Dicke der erforderlichen Dispersions-Kompensation entsprechend angepasst werden.

In den bisher beschriebenen Anordnungen hat man immer noch alle zu den verschiedenen Objekttiefen gehörigen Messstrahlen gleichzeitig am Photodetektor, weil die Reflektoren 517 und 519 teildurchlässig sein müssen. Das vermindert die Signalqualität und kann zu Signalverwechslungen führen. Die nachfolgend beschriebene weitere Ausformung der erfindungsgemäßen Anordnung vermeidet diese Probleme.

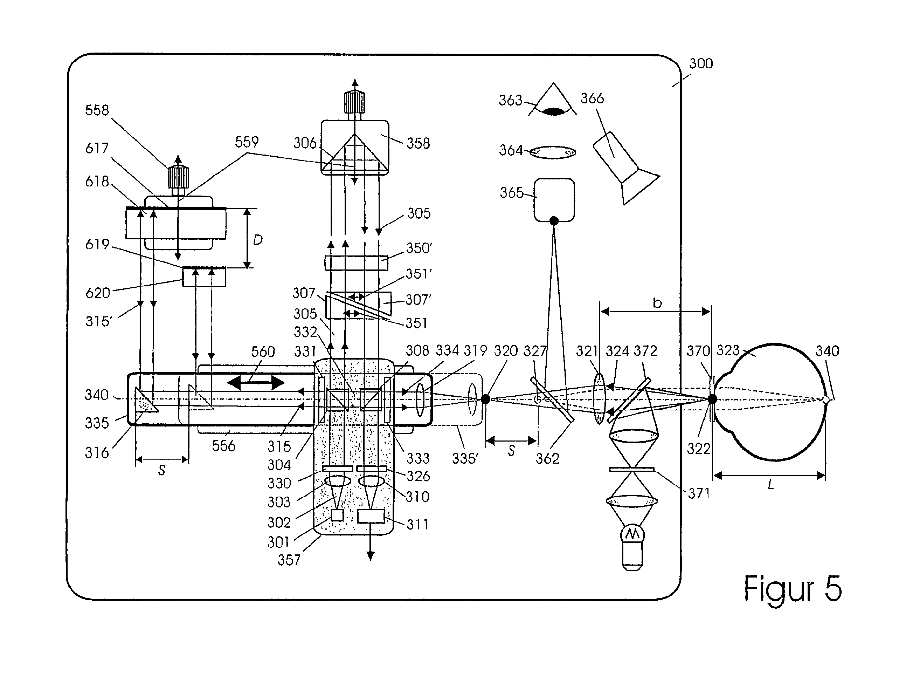

In der Anordnung nach Figur 5 trifft der von dem 90-Grad-Spiegel 316 um 90° aus seiner ursprünglichen Richtung reflektierte Messstrahl 315 je nach Position der Scannerplatte 335 auf mehrere nicht nur in der Tiefe, sondern auch seitlich gestaffelte Reflektoren 617 (mit Platte 618) und 619 (mit Platte 620). Das hat zur Folge, dass jeweils nur ein einziger Messstrahl an der kurzkohärenz-interferometrischen Messung teilnimmt. Hier können alle Reflektoren voll verspiegelt ausgeführt sein. Alle in der Tiefe separierten Messungen werden nun optisch getrennt ausgeführt. Auch hier braucht die Scannerplatte 335 zur Messung der Augenlänge $L$ nur eine Distanz von etwa $S = L - D$ bewegt zu werden.

Auch hier muß der Abstand des Corneascheitels von der Relais-Optik 321 gleich $b = f + \frac{f^2}{L-D}$ betragen.

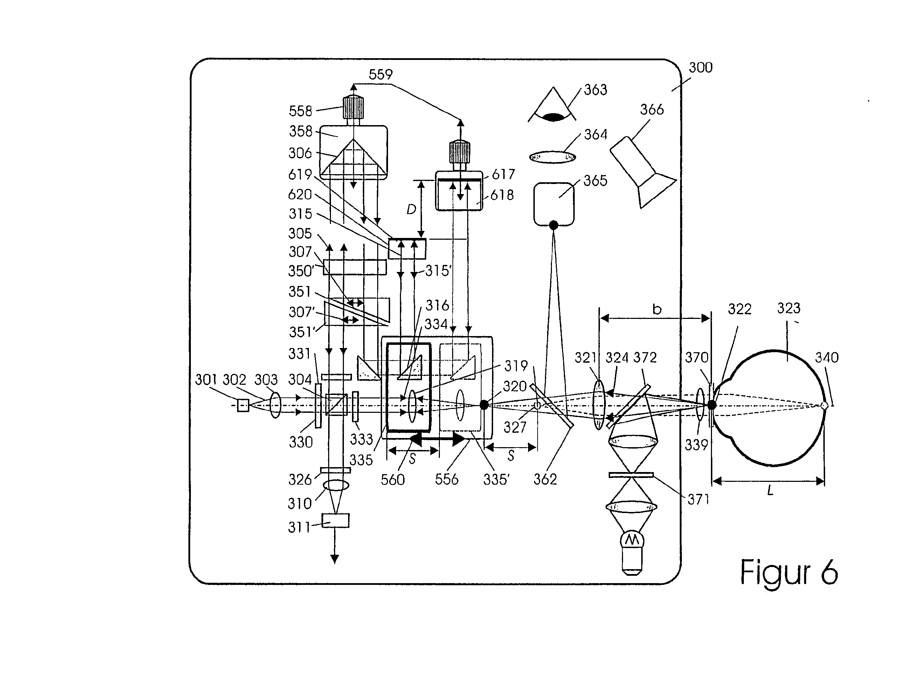

Die Figur 6 skizziert einen vereinfachten Interferometeraufbau. Hier sind 90-Grad-Spiegel 316 und Fokussieroptik 319 nebeneinander auf der Scannerplatte 335 montiert, die sich in Richtung der optischen Achse 340 periodisch zwischen der durchgezogen gezeichneten Position 335 und der gestrichelt gezeichneten Position 355' hin und her bewegt (Doppelpfeil 560). Die übrigen Bezeichnungen entsprechen denen der vorhergehenden Figuren. Hier erübrigt sich die Trägerplatte 357.

Auch alle anderen im Zusammenhang mit den Figuren 3, 4 und 5 erläuterten Vorrichtungen, wie beispielsweise die Vermeidung von störenden Reflexionen durch polarisationsoptische Massnahmen und die Dispersionskompensation sind für die Anordnung nach Figur 6 ausführbar. Bei Fehlsichtigkeit kann dem Auge 323 eine sammelnde oder zerstreuende Hilfsoptik 339 vorgesetzt werden, welche die Fehlsichtigkeit kompensiert.

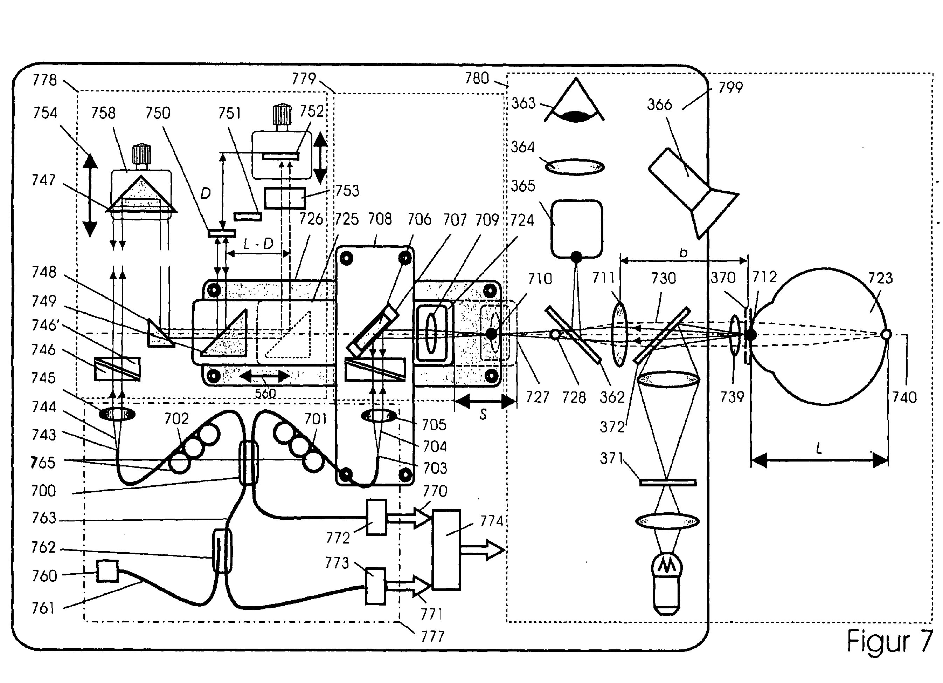

Faseroptische Implementierungen der erfindungsgemäßen Anordnungen. Eine vorteilhafte Implementierung der vorliegenden Erfindung erfolgt auf Basis faseroptischer Interferometer. Erfindungsgemäß ausgestaltete Referenz- und Mess-Interferometerarme werden mit den Armen eines faseroptischen Michelson-Interferometers kombiniert. Es ergeben sich Vorteile, weil die zentrale Interferometerstruktur mit den faseroptischen Strahlteilern kompakt, robust gegenüber Erschütterungen und betriebssicher ist. Es sei darauf hingewiesen, dass es verschiedene Arten gibt, das faseroptische Michelson-Interferometer insbesondere auf der Detektorseite auszugestalten. In Figur 7 ist (in dem strichpunktierten Kasten 777) ein faseroptisches Interferometer mit dem Stand der Technik entsprechender "Balanced Detection" skizziert. "Balanced Detection" kompensiert das Modenrauschen der hier erforderlichen breitbandigen Lichtquellen, was einen Signal-zu-Rauschen Gewinn von bis zu 20 dB ermöglicht. Die Erfindung betrifft hier nicht diese faseroptischen Interferometer und die zugehörige Signalverarbeitung. Es können auch andere faseroptische Interferometerstrukturen benutzt werden, beispielsweise auch solche, die faseroptische Zirkulatoren verwenden. Vielmehr betrifft die Erfindung die optomechanische Struktur und Implementierung der an die Faserinterferometer-Ausgänge 703 und 743 angekoppelten optischen Strahlengänge. Es können die Ausgänge 703 und 743 verschiedener faseroptischer Interferometer benutzt werden. Auch kann die Signalverarbeitung unterschiedlich erfolgen. So kann beispielsweise das elektrische Ausgangssignal den direkten Zeitverlauf des Interferenzterms wiedergeben oder es kann demoduliert werden, so dass nur die Einhüllende am Ausgang erscheint.

Der Kern des abgebildeten faseroptischen Michelson-Interferometers wird von einem Faserkoppler 700 gebildet, der das von der Kurzkohärenz-Lichtquelle 760 kommende Licht auf Interferometer-Messarm 701 und Interferometer-Referenzarm 702 aufteilt. Das bei 703 aus der Faser austretende Lichtbündel 704 wird von der Optik 705 eines Faser-Kollimators kollimiert und von dem Spiegel 706 in Richtung optischer Achse 740 des Kurzkohärenz-Interferometers gerichtet. Der Reflektor 706 ist zur leichteren Justierbarkeit in einer um zwei in seiner Spiegelebene liegende Achsen drehbaren Halterung 707 montiert. Die Halterung 707 ist fest auf der Platte 708 montiert, die ihrerseits fest mit der Grundplatte 799 verbunden ist und die Scannerplatte 725 überbrückt. Das vom Spiegel 706 in Richtung der optischen Achse 740 reflektierte Lichtbündel 704 wird von der Fokussieroptik 709 in den Fokus 710 fokussiert. Der Fokus 710 wird von der Relais-Optik 711 in den Punkt 712 abgebildet. Der Punkt 712 befindet sich auf der Cornea eines Auges 723, dessen Länge L gemessen wird.

Die Fokussieroptik 709 ist mittels einer Halterung 724 auf der Scannerplatte 725 montiert. 725 kann der bewegte Schlitten eines Voice Coil Scanners - beispielsweise der Firma Physik Instrumente - oder eines Ultraschall-Scanningtisches oder einer anderen entsprechenden Vorrichtung sein, dessen Fußplatte 726 auf der Grundplatte 799 befestigt ist. Während der Messung wird die Scannerplatte 725 entlang der optischen Achse 740 um die Strecke S zwischen der durchgezogen gezeichneten Position 725 und der gestrichelt gezeichneten Position 727 periodisch hin und her bewegt. Befindet sich die Scannerplatte in der gestrichelt gezeichneten Position, dann befindet sich der Fokus des Lichtbündels 704 - beispielsweise in der in Figur 7 dargestellten Konfiguration - im Brennpunkt 728 der Relais-Optik 711 und das Lichtbündel 704 wird als parallel kollimiertes Lichtbündel 730 auf das Auge 723 gerichtet. Das Auge fokussiert dieses Lichtbündel auf seinen Fundus. Bei Fehlsichtigkeit kann dem Auge 723 eine sammelnde oder zerstreuende Hilfsoptik 739 vorgesetzt werden, welche die Fehlsichtigkeit kompensiert.

Das bei 743 aus der Lichtleitfaser 702 austretende Referenzlichtbündel 744 wird von der Optik 745 eines Faser-Kollimators kollimiert und nach Durchlaufen einer beispielsweise aus zwei Keilplatten (746, 746') bestehenden Dispersionskompensations-Vorrichtung von dem Dachkantspiegel 747 auf den 90-Grad-Spiegel 748 und von diesem in Richtung optischer Achse 740 des Kurzkohärenz-Interferometers gerichtet. Das Lichtbündel 744 trifft dort auf den bewegten 90-Grad-Spiegel 749, der das Lichtbündel 744 auf den Reflektor 750 richtet. Das Referenzlichtbündel 744 wird von diesem Spiegel in sich selbst reflektiert und nimmt seinen Weg zurück bis in die Lichtfaser 702.

Wenn sich die Scannerplatte 725 entlang der optischen Achse 740 in Richtung auf die gestrichelt gezeichnete Position hin bewegt, wird das Referenzlichtbündel 744 von dem 90-Grad-Spiegel 749 hintereinander auf die Reflektoren 750, 751 und 752 gerichtet. Es können hier noch weitere Reflektoren in der Tiefe und seitlich gestaffelt angeordnet werden. Außerdem können hier auch Planplatten 753 zur Dispersions-Kompensation angebracht werden. So werden Referenzlicht-Wegstrecken unterschiedlicher Längen realisiert, beispeisweise um - neben der Position von Fundus und Corneascheitel - Positionen von weiteren Strukturen wie der inneren Corneafläche, der Linsenvorderfläche und der Linsenrückfläche zu messen.

In der Figur 7 ist beispielsweise der Fall skizziert, bei dem der Abstand zwischen den (Cornea - und Fundus-assoziierten) Reflektoren 750 und 752 gleich der Strecke D ist. Wie schon im Zusammenhang mit der Anordnung nach Figur 4 beschrieben, verkleinert sich so die von der Scannerplatte 725 zur Augenlängenmessung zurückzulegende Strecke um D. Zur Messung der Länge L eines Auges, bedarf es nur einer Bewegung um die Strecke S = L - D. Tatsächlich könnte man diese Strecke sogar fast Null machen: man bräuchte die Scannerplatte 725 ja eigentlich nur um die Kohärenzlänge $l_C$ bewegen - um die Interferenz nachzuweisen. Wegen der aber doch recht großen Streuung tatsächlicher Augenlängen wird man die Scannerplatte 725 um eine Strecke von etwa dieser Streuung, also um einige (x) Millimeter, bewegen müssen. Jedenfalls kann die ansonsten doch recht lange zu scannende Strecke L auf wenige Millimeter reduziert werden. Entsprechend breit müssen die Reflektoren 750, 751, 752 sein: nämlich gleich der Breite des Strahls 744 plus x Millimeter. Eine weitere Anpassung ist durch die Justierung der Referenzstrahllänge mittels des Reflexionsprismas 747 möglich, das auf einer Verstellvorrichtung 758 montiert ist, die in die Richtungen des Doppelpfeils 754 verschiebbar ist.

Wie oben angedeutet, ist erfindungsgemäß vorgesehen, dass das Messlichtbündel 704 in den Positionen 725 und 727 der Scannerplatte einmal auf die Cornea fokussiert wird und einmal am Fundus. Damit bei einer Verschiebung der Scannerplatte 725 um die Strecke S = L - D der Messstrahl 704 einmal an der Cornea (durchgezogen gezeichnete Scannerplatten-Position) und einmal am anderen Ende der Bewegung (gestrichelt gezeichnete Scannerplatten-Position) fokussiert wird muß, wie schon im Zusammenhang mit der Figur 3 erläutert, die Distanz des Scheitels der Vorderfläche der Cornea von der Relais-Optik 711 gleich $b = f\left(1 + \dfrac{f}{L-D}\right)$ sein; *f* ist die Brennweite der Relais-Optik 711. Falls sich das Kohärenzfenster anfangs (Scannerplatten-Position 725) an der Cornea befindet, wird es sich am Schluß der Verschiebung der Scannerplatte (Scannerplatten-Position 727) am Fundus befinden. Ist beispielsweise $S = f$, dann ist $b = 2f$; für eine Brennweite der Relais-Optik 711 von $f = 50mm$ erhält man $b = 100mm$.

Es sei erwähnt, dass man den tatsächlichen mechanischen Scanbereich der Scannerplatte 725 etwas größer als S wählen wird, beispielsweise um im eigentlichen Messbereich eine annähernd konstante Geschwindigkeit zu realisieren, was die nachfolgende elektronische Signalverarbeitung erleichtert.

Ferner kann die Position des Reflexionsprismas 747 mit Hilfe des Verschiebetisches 758 so abgestimmt werden, dass der Fokus des Messstrahls am Auge für einen Punkt etwa in der Mitte der Vorderkammer auch in der Mitte des Kohärenzfensters liegt. Eine solche Justierung ist besonders dann von Interesse, wenn es um präzise Messungen der Vorderkammergeometrie geht.

Auch hier können mittels eines weiteren Reflektors 751, wie schon im Zusammenhang mit dem Reflektor 519 in der Figur 4 erläutert, beispielsweise die Vorderkammertiefe oder andere Teilstrecken des Auges gemessen werden.

Als Kurzkohärenz-Lichtquelle 760 kann eine mit einer "Pig-Tail" Faser ausgestattete Superlumineszenzdiode oder eine andere Kurzkohärenz-Lichtquelle verwendet werden, wobei die Strahlung letzterer in die Lichtleitfaser 761 mittels dem Stand der Technik entsprechender Koppler einzukoppeln ist. Bei Verwendung einer Pig-Tail Superlumineszenzdiode kann die Strahlung aus dem Pig-Tail direkt in den ersten Faserkoppler 762 eingekoppelt werden. Dieser koppelt die Lichtwelle in die Faser 763, die die Lichtwelle im 50:50 Koppler (3dB-Koppler) 700 auf Messfaser 701 und Referenzlichtfaser 702 verteilt. Faserschleifen-Polarisations-Controller 765 können zur Einstellung des Polarisationszustands in den zwei Interferometerarmen verwendet werden. Die elektrischen Ausgänge 770 und 771 der beiden Photodetektoren 772 und 773 werden an die Eingänge eines Differentialverstärkers 774 gelegt, dessen Ausgangssignal beispielsweise bandpassgefiltert und demoduliert wird.

Zur Beobachtung der Position des Probandenauges 723 relativ zum Messstrahl kann - wie schon im Zusammenhang mit der Anordnung nach Figur 3 beschrieben - auch hier im Messstrahl ein teildurchlässiger Spiegel 362 angeordnet werden. Die Beobachtung kann dann direkt (363), mit Hilfe eines Okulars 364 oder mit Hilfe einer Fernsehkamera 365 erfolgen. Es kann auch hier sinnvoll sein, das Probandenauge 323 zusätzlich mit einer inkohärenten Lichtquelle 366 zu beleuchten. Ferner kann auch hier zur präzisen Positionierung des Probandenauges auf der Interferometerachse 740 das Bild 370 einer Strichplatte 371 benutzt werden, welches über einen teildurchlässigen Spiegel 372 auf die Probanden-Cornea gespiegelt wird.

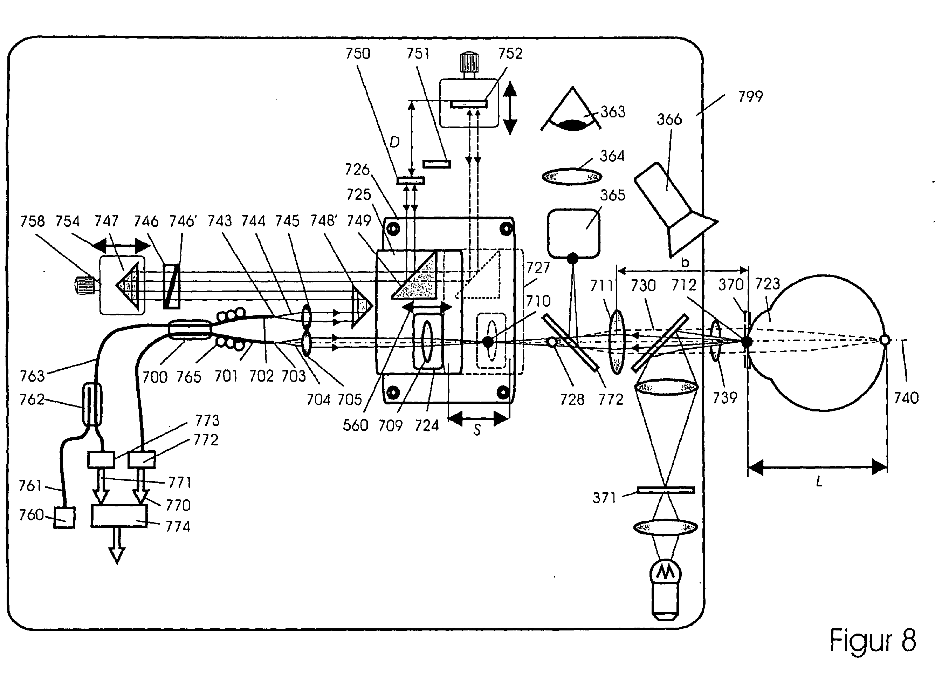

In der Figur 7 sind noch 3 weitere Kästen (gestrichelt) eingezeichnet: 778, 779 und 780. Die in diesen Kästen befindlichen Bauteile bilden Funktionsgruppen, die weitgehend separat aufgestellt werden können. Der Strahlengang, wie in der Figur 8 angegeben, ist zunächst gegenüber jenem der Figur 7 dadurch verschieden, dass der zum Referenzstrahlengang gehörende bewegte 90-Grad-Spiegel 749 und die den Fokus 710 im Messstrahlengang erzeugende Fokussieroptik 709 auf der Scannerplatte nebeneinander angeordnet sind. Damit entfällt die Platte 708 und alle auf ihr befmdlichen Komponenten können direkt auf der Grundplatte 799 montiert werden, was den Aufbau nicht nur insgesamt vereinfacht sondern auch stabiler macht.

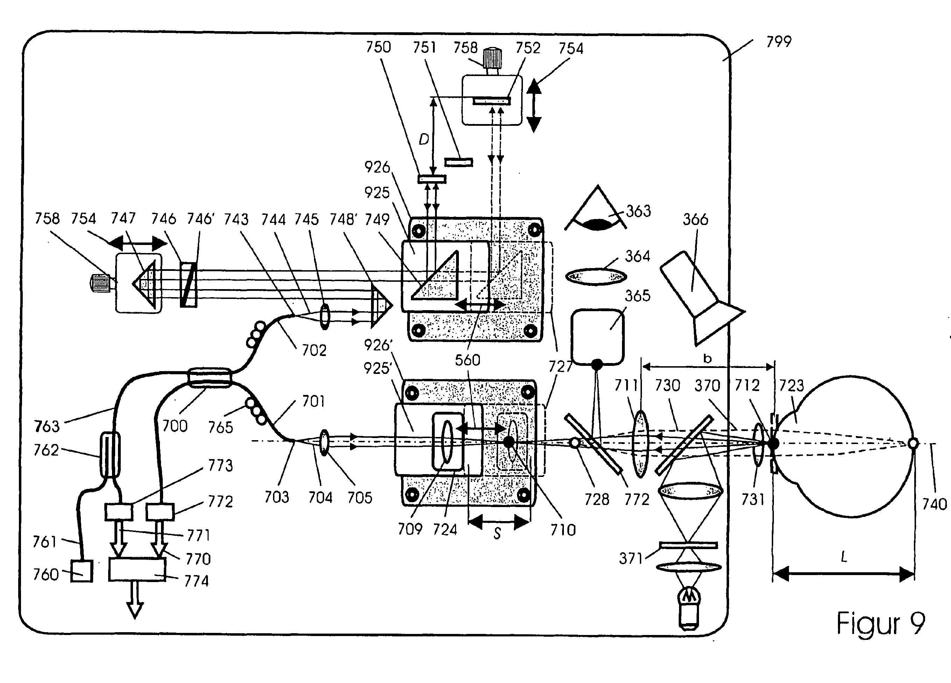

Schließlich ist in der Figur 9 ein weiterer erfindungsgemäßer Strahlengang angegeben, bei dem der bewegte 90-Grad-Spiegel 749 des Interferometer-Referenzarms und die bewegte Fokussieroptik 709 des Interferometer-Messarms auf separaten Scannerplatten 925 und 925' montiert sind. Diese separaten Scannerplatten können elektrisch synchronisiert werden. Sie können jedoch auch mit unterschiedlichen Scan-Strecken betrieben werden. Es muß lediglich sichergestellt werden, dass $S \geq L - D$ und die Distanz des Scheitels der Vorderfläche der Cornea von der Relais-Optik 711 gleich $b = f\left(1 + \dfrac{f}{L-D}\right)$ ist, mit der Brennweite *f* der Relais-Optik 711. Über die geeignete Wahl der Brennweite *f* der Relais-Optik 711 kann das ophthalmolgische Kurzkohärenz-Interferometer - bei sonst unveränderten Parametern - leicht an andere ophthalmologische Einrichtungen und Messvorrichtungen angebaut werden. Anstelle des hier dargestellten Interferometer-Referenzarms kann als Referenzarm auch eine sogenannte "Rapid Scan Optical Delay Line", wie in K. F. Kwong, et al.: 400-Hz mechanical scanning optical delay line, Optics Letters 18 (1993) pp. 558-560, beschrieben, mit dem Interferometer-Meßarm nach Figur 9 kombiniert werden.

Die im Zusammenhang mit der Anordnung nach Figur 3 beschriebenen Modifizierungen zur Vermeidung von störenden Reflexionen, zur Dispersionskompensation und zur Beobachtung der Position des Probandenauges lassen sich auch in dem Strahlengang nach Figur 7, 8 und 9 sinngemäß realisieren. Man kann ferner die Position des Reflektors 752 und das Dachkantprisma 747 mit Hilfe der Verschiebetische 758 durch axiales Verschieben (Doppelpfeile 754) so auf die aktuellen Teilstreckenlängen des Auges abstimmen, dass der erforderliche mechanische Scanvorgang des Kurzkohärenz-Interferometers auf wenige Millimeter reduziert wird. Dadurch wird der Messvorgang am Auge nicht nur erheblich beschleunigt, sondern auch der Einsatz billigerer Scanner ermöglicht.

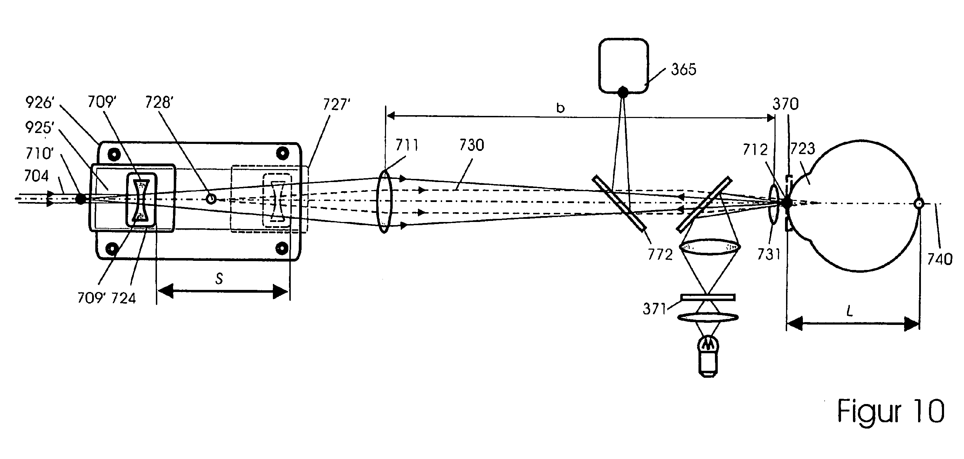

Schließlich sei auch noch bemerkt, dass als Fokussieroptik (103, 319, 709) auch eine Zerstreuungslinse (709') verwendet werden kann. Die Figur 10 skizziert einen entsprechenden Strahlengang des Interferometer-Meßarms. An die Stelle des reellen Fokus 728 tritt nun der virtuelle Fokus 728.

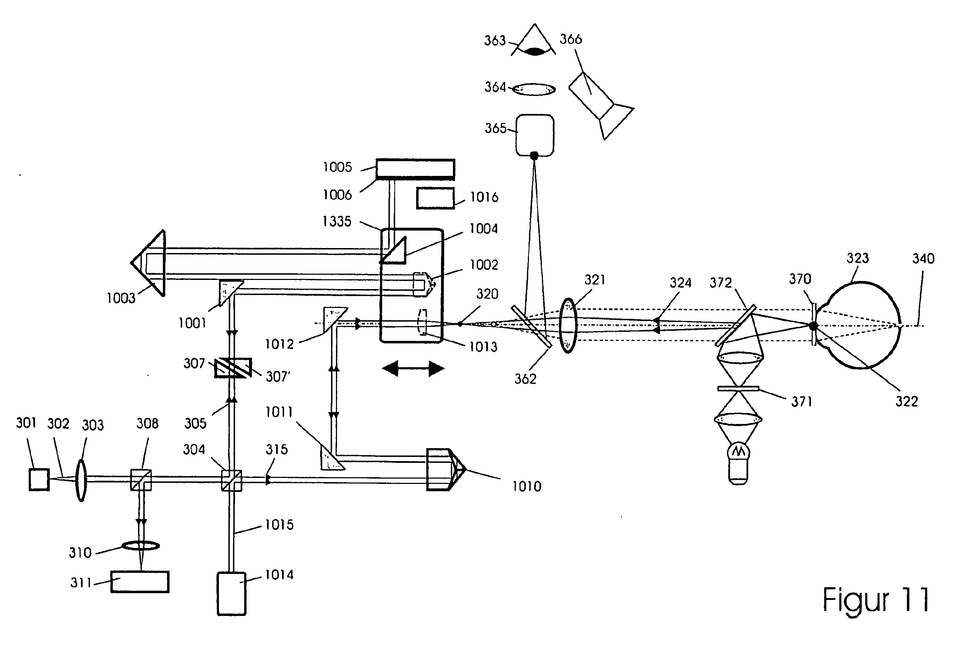

Eine weitere erfindungsmäßige Anordnung ist in der Figur 11 dargestellt. Diese Anordnung erlaubt es, durch Faltung des Referenzstrahls Scanner mit kleinerem Scan-Hub zu verwenden. In der gezeichneten Anordnung läuft der Referenzstrahl 3-mal zwischen den Reflektoren 1001, 1002, 1003 und 1004 hin und her. So ergibt sich eine Verkleinerung des Scannerhubs um den Faktor 3. Mit noch mehr Reflektoren kann eine noch weitere Verkürzung des erforderlichen Scan-Hubs erreicht werden.

Die Kurzkohärenz-Lichtquelle 301, beispielsweise eine Superlumineszenzdiode, emittiert einen zeitlich teilkohärenten und räumlich möglichst vollständig kohärenten Lichtstrahl 302, der mittels einer Optik 303 durch den Strahlteiler 308 hindurch den Strahlteiler 304 beleuchtet. Der Strahlteiler 304 teilt den Lichtstrahl 302 in Messstrahl 315 und Referenzstrahl 305. Der Referenzstrahl 305 wird von einer Reihe von Spiegeln und Reflektoren optisch gefaltet: Der Referenzstrahl 305 wird zunächst von dem 90°-Spiegel 1001 nach Durchlaufen der Dispersions-Kompensationsprismen 307 und 307' auf den Retroreflektorspiegel 1002 und von diesem zurück auf den Reflektorspiegel 1003 gerichtet, der den Referenzstrahl weiters auf den 90°-Spiegel 1004 reflektiert. Der 90°-Spiegel 1004 richtet den Referenzstrahl 305 schließlich auf den Referenzspiegel 1005 mit der Spiegelfläche 1006. Der dort reflektierte Referenzstrahl läuft durch die optische Faltung zurück und wird von dem Strahlteiler 308 und der Optik 310 auf den Photodetektor 311 gerichtet.

Der vom Strahlteiler 304 (in der Zeichnung nach rechts) transmittierte Messstrahl 315 wird von dem Retroreflektor 1010 über die 90-Grad-Spiegel 1011 und 1012 auf die Fokussieroptik 1013 und von dieser in den Fokus 320 fokussiert. Die Relais-Optik 321 bildet den Fokus 320 auf das Auge 323 den Punkt 322 ab. Der Punkt 322 befindet sich auf der Cornea des Auges 323, dessen Länge beispielsweise gemessen wird.

Die Optiken 1013, 1002 und 1004 sind auf einer in Richtung der strichpunktiert gezeichneten Interferometerachse 340 beweglichen Scannerplatte 1335 montiert. Die Scannerplatte 1335 kann der bewegte Schlitten eines Voice Coil Scanners - beispielsweise der Firma Physik Instrumente - oder eines Ultraschall-Piezo-Scanningtisches oder einer anderen entsprechenden Vorrichtung sein.

Weiters ist hier ein Hilfslaser 1014 für Justierzwecke, beispielsweise eine Helium-Neon-Laser, angeordnet, dessen Strahl 1015 über den Strahlteiler 304 eingespiegelt wird. Die Planplatte 1016 dient zur Dispersionskompensation für Messungen in der Tiefe des Auges 323.

Für alle Anordnungen kann auch die Ausgestaltung in einer Dualbeamanordnung verwendet werden, mit dem bekannten Vorteil, die Augenbewegungen des Probanden entsprechend kompensieren zu können.

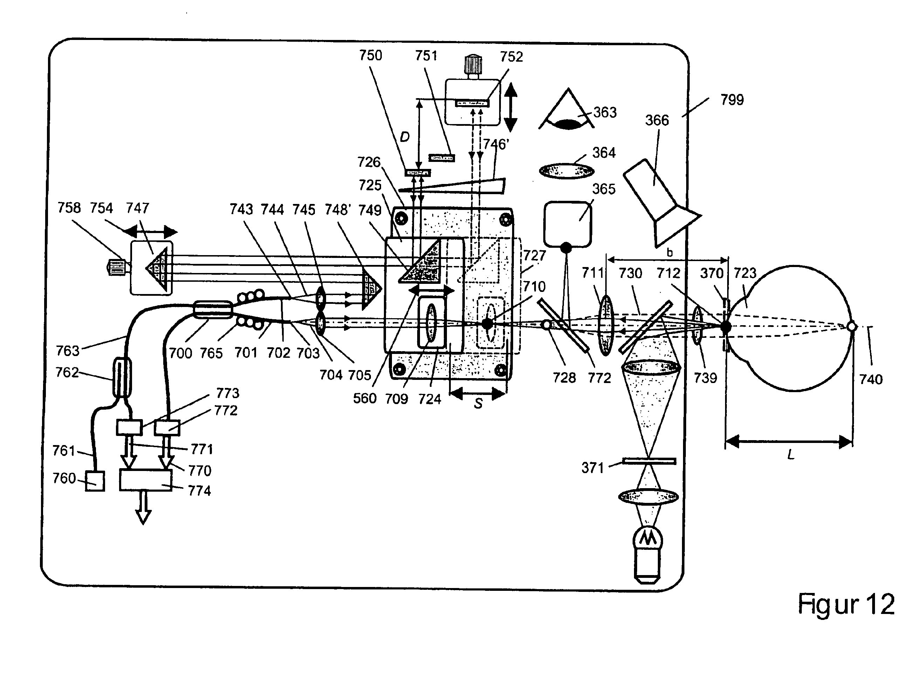

Die Dispersionskompensation kann wie am Beispiel in Figur 12 dargestellt auch durch eine Keil - oder Prismenanordnung 746, die abhängig von der Verschiebeposition des Scanningtisches durchlaufen wird, ausgeführt werden. Die dabei auftretenden Strahlablenkungen (nicht dargestellt) sind bei der Ausrichtung der übrigen Bauelemente (z.B. Referenzspiegel) zu berücksichtigen.

Eine weitere vorteilhafte Ausgestaltung des Aufbaus besteht darin, zusätzliche Mittel vorzusehen, die es erlauben die gezielte Ausrichtung der optischen Achse der Vorrichtung gegenüber der optischen Achse des Auges bzw. der Sehachse vornehmen zu können, beispielsweise durch ein Verfahren wie in der PCT-Anmeldung WO 02/065899 A2, auf deren gesamten Inhalt hiermit Bezug genommen wird, beschrieben oder durch entsprechend verstellbare Strahlablenkelemente (Prismen, Keile) in der Vorrichtung selbst.

**Patentansprüche**

1. Kurzkohärenz-Interferometer zur Messung von Teilstrecken des Auges, das den Messstrahl auf das jeweilige zugeordnete Kohärenzfenster fokussiert und / oder die erforderlichen Interferometerspiegel-Scan-Strecken auf kleinere Strecken als die zu messenden Distanzen reduziert, wobei im Kurzkohärenz-Interferometer mindestens ein Umlenkelement (z.B. Spiegel, Prisma) mit einem Umlenkwinkel a (316, 749) und Elemente einer Fokussieroptik (319, 709) vorgesehen sind, welche eine periodische Hin-und-her Bewegung (Doppelpfeil 560) so ausführen, dass der von der Fokussieroptik erzeugte und mittels einer Relais-Optik (103, 321, 711) auf das Auge abgebildete Messstrahlfokus (101', 322, 712) synchron mit dem Kohärenzfenster von der Cornea entlang der optischen Achse des Auges bis zur Retina (z.B. Fovea centralis) und zurück bewegt wird, **gekennzeichnet dadurch, dass** das mindestens eine Umlenkelement während seiner Bewegung den Mess- oder Referenzstrahl sequentiell auf eine Reihe von in der Tiefe und / oder seitlich gestaffelten Reflektoren richtet.

2. Kurzkohärenz-Interferometer zur Messung der Teilstrecken des Auges nach Anspruch 1, **dadurch gekennzeichnet,**

**dass** die in der Tiefe und/oder seitlich gestaffelten Reflektoren in ihrer Position einstellbar sind und/oder die Positionierung adaptiv nach vorab ermittelten Sollpositionen der Augengrenzflächen erfolgt

3. Kurzkohärenz-Interferometer zur Messung der Teilstrecken des Auges nach einem der Ansprüche 1 bis 2, **dadurch gekennzeichnet, dass** mindestens ein Umlenkelement (316, 749) und die Fokussieroptik (319, 709, 709') auf einem periodisch hin und her bewegten Tisch einer Scanningvorrichtung in Bewegungsrichtung hintereinander und/oder nebeneinander angebracht sind.

4. Kurzkohärenz-Interferometer zur Messung der Teilstrecken des Auges nach einem der Ansprüche 1 bis 2, **dadurch gekennzeichnet, dass** mindestens ein Umlenkelement (316, 749) und die Fokussieroptik (319, 709, 709') auf dem periodisch hin und her bewegten Tisch einer Scanningvorrichtung unter einem beliebigen Winkel zur Bewegungsrichtung hintereinander und/oder nebeneinander angebracht sind.

5. Kurzkohärenz-Interferometer zur Messung der Teilstrecken des Auges nach einem der vorigen Ansprüche, **dadurch gekennzeichnet, dass** der von der Fokussieroptik (319, 709, 709') erzeugte Messstrahlfokus (101, 320, 710, 710') von einer Relais-Optik (321, 711) auf das Auge abgebildet wird, wobei der Corneascheitel genau oder annähernd im Abstand $b = f\left(1 + \dfrac{f}{L-D}\right)$ von der Relais-Optik angeordnet wird, mit $f$ = Brennweite der Relaisoptik, $L$ = optische Länge des Auges, $D$ = Abstand der zu Cornea (317, 617, 750) und Fovea centralis (517, 619, 752) gehörenden Reflektoren.

6. Kurzkohärenz-Interferometer zur Messung der Teilstrecken des Auges nach einem der vorigen Ansprüche, **dadurch gekennzeichnet, dass** der von der Fokussieroptik (319, 709, 709') erzeugte Messstrahlfokus (320, 710, 710') um eine Strecke etwas größer als $L - D$ ($L$ = optische Länge des Auges; $D$ = Distanz der zu Cornea und Fovea centralis gehörenden Reflektoren) periodisch hin und her bewegt wird.

7. Kurzkohärenz-Interferometer zur Messung der Teilstrecken des Auges nach einem der vorigen Ansprüche, **dadurch gekennzeichnet, dass** die Aufspaltung in die interferometrischen Mess- und Referenzarme (701, 702) des Kurzkohärenz-Interferometers mittels eines oder mehrerer faseroptischer Koppler (700) erfolgt.

8. Kurzkohärenz-Interferometer zur Messung der Teilstrecken des Auges nach Anspruch 1, **dadurch gekennzeichnet, dass** mindestens ein Umlenkelement (316, 749) und Elemente der Fokussieroptik (319, 709, 709') auf getrennten periodisch hin und her bewegten Scanningvorrichtungen in Bewegungsrichtung oder unter einem Winkel hierzu nebeneinander montiert sind.

9. Kurzkohärenz-Interferometer zur Messung der Teilstrecken des Auges nach Anspruch 1, **dadurch gekennzeichnet, dass** mindestens ein Umlenkelement (316, 749) und Elemente der Fokussieroptik (319, 709, 709') auf getrennten periodisch hin und her bewegten Scanningvorrichtungen montiert sind und die Bewegungen der zwei Scanningvorrichtungen elektronisch synchronisiert werden, oder die Bewegungen relativ zueinander funktionell modifiziert werden können

10. Kurzkohärenz-Interferometer zur Messung der Teilstrecken des Auges nach einem der vorigen Ansprüche, **dadurch gekennzeichnet, dass** die Scanningvorrichtung (355 mit 356, 725 mit 726, sowie 925 und 925' mit 926 und 926') vorzugsweise ein Schrittmotor- oder Piezomotor-gesteuerter Scanningtisch, ein Voice Coil Scanner oder ein Ultraschall-Piezo-Scanningtisch ist.

11. Kurzkohärenz-Interferometer zur Messung der Teilstrecken des Auges nach einem der vorigen Ansprüche, **dadurch gekennzeichnet, dass** als Fokussieroptik (319, 709) eine Sammellinse, eine Zerstreuungslinse (709') oder ein optisches System aus mehreren, festen oder variablen Elementen verwendet wird.

12. Kurzkohärenz-Interferometer zur Messung der Teilstrecken des Auges nach einem der vorigen Ansprüche, **dadurch gekennzeichnet, dass** im Referenz- oder Messarm auch eine sogenannte "Rapid Scan Optical Delay Line" oder ein anderer Weglängenmodulator mit dem Interferometer-verwendet werden.

13. Kurzkohärenz-Interferometer zur Messung der Teilstrecken des Auges nach einem der vorigen Ansprüche, **dadurch gekennzeichnet, dass** mit zusätzlichen Mitteln zur Verstellung eines optischen Elementes im Strahlengang, z.B.

mittels eines Spiegels (306, 747) die Anfangskoinzidenz von Meßfokus und Kohärenzfenster etwa in die Mitte der Vorderkammer des Auges oder an eine andere beliebige Stelle gelegt wird.

14. Kurzkohärenz-Interferometer zur Messung der Teilstrecken des Auges nach einem der vorigen Ansprüche, **dadurch gekennzeichnet, dass** der Scan-Hub durch optische Faltung des Referenz- und /oder Messstrahlengangs verkleinert wird.

15. Kurzkohärenz-Interferometer zur Messung der Teilstrecken des Auges nach einem der vorigen Ansprüche, **dadurch gekennzeichnet, dass** eine Dispersionskompensation automatisch erfolgt, indem im Referenzstrahlengang parallel zur Bewegungsrichtung angeordnete Keilplatten durchlaufen werden und die Kompensationswirkung damit von der Verschiebeposition abhängt.

16. Kurzkohärenz-Interferometer zur Messung der Teilstrecken des Auges nach Anspruch 1, **dadurch gekennzeichnet, dass** Mittel zur Verstellung oder Orientierung der Meßstrahlachse zur optischen Achse bzw. der Sehachse des Auges vorgesehen sind.

17. Kurzkohärenz-Interferometer zur Messung der Teilstrecken des Auges nach einem der vorigen Ansprüche, **dadurch gekennzeichnet, dass** ein Aufbau nach dem Dualbeam Verfahren verwendet wird.

18. Kurzkohärenz-Interferometer zur Messung der Teilstrecken des Auges nach einem der vorigen Ansprüche, **dadurch gekennzeichnet, dass** als Umlenkelement ein 90-Grad-Spiegel oder -Prisma vorgesehen ist.

**Claims**

1. Short coherence interferometer for measuring partial distances of an eye which focuses the measurement beam onto the respective assigned coherence window, and/or reduces the required interferometer mirror scanning paths to shorter paths than the distances to be measured, and being provided in the short coherence interferometer at least one deflecting element (for example mirror, prism) with a deflection angle $\alpha$ (316, 749), and elements of a focusing optics (319, 709) which execute a periodic back and forth movement (double arrow 560) such that the measurement beam focus (101', 322, 712) produced by the focusing optics and imaged onto the eye by means of a relay optics (103, 321, 711) is moved synchronously with the coherence window from the cornea along the optical axis of the eye up to the retina (for example fovea centralis) and back, **characterized in that** during its movement the at least one deflecting element directs the measurement or reference beam sequentially onto a row of reflectors which are staggered in depth and/or laterally.

2. Short coherence interferometer for measuring the partial distances according to Claim 1, **characterized in that** the reflectors staggered in depth and/or laterally can have their position set, and/or the positioning is performed adaptively in accordance with previously determined desired positions of the boundary surfaces of the eye.

3. Short coherence interferometer for measuring the partial distances of the eye according to either of Claims 1 and 2, **characterized in that** at least one deflecting element (316, 749) and the focusing optics (319, 709, 709') are mounted behind one another and/or next to one another in the movement direction on a table, that is moved periodically back and forth, of a scanning device.

4. Short coherence interferometer for measuring the partial distances of the eye according to either of Claims 1 and 2, **characterized in that** at least one deflecting element (316, 749) and the focusing optics (319, 709, 709') are mounted behind one another and/or next to one another on the table, that is moved periodically back and forth, of a scanning device, at any desired angle to the movement direction.

5. Short coherence interferometer for measuring the partial distances of the eye according to one of the preceding claims, **characterized in that** the measurement beam focus (101, 320, 710, 710') produced by the focusing optics (319, 709, 709') is imaged onto the eye by a relay optics (321, 711), the corneal apex being arranged exactly or approximately at the distance $b = f\left(1 + \dfrac{f}{L-D}\right)$ from the relay optics, where $f$ = focal length of the relay optics, $L$ = optical length of the eye, $D$ = distance of the reflectors belonging to cornea (317, 617, 750) and fovea centralis (517, 619, 752).

6. Short coherence interferometer for measuring the partial distances of the eye according to one of the preceding claims, **characterized in that** the measurement beam focus (320, 710, 710') produced by the focusing optics (319, 709, 709') is moved periodically back and forth along a path somewhat larger than $L - D$ ($L$ = optical length of the eye; $D$ = distance of the reflectors belonging to cornea and fovea centralis).

7. Short coherence interferometer for measuring the partial distances of the eye according to one of the preceding claims, **characterized in that** the splitting into the interferometric measurement and reference arms (701, 702) of the short coherence interferometer is performed by means of one or more fibre-optic couplers (700).

8. Short coherence interferometer for measuring the partial distances of the eye according to Claim 1, **characterized in that** at least one deflecting element (316, 749) and elements of the focusing optics (319, 709, 709') are mounted next to one another on separate scanning devices, that are moved periodically back and forth, in the movement direction or at an angle thereto.

9. Short coherence interferometer for measuring the partial distances of the eye according to Claim 1, **characterized in that** at least one deflecting element (316, 749) and elements of the focusing optics (319, 709, 709') are mounted on separate scanning devices, that are moved periodically back and forth, and the movements of the two scanning devices are electronically synchronized, or the movements can be modified functionally relative to one another.

10. Short coherence interferometer for measuring the partial distances of the eye according to one of the preceding claims, **characterized in that** the scanning device (355 with 356, 725 with 726, as well as 925 and 925' with 926 and 926') is preferably a scanning table controlled by a step-up motor or piezo motor, a voice coil scanner or an ultrasound piezo scanning table.

11. Short coherence interferometer for measuring the partial distances of the eye according to one of the preceding claims, **characterized in that** a converging lens, a diverging lens (709') or an optical system composed of a plurality of fixed or variable elements is used as focusing optics (319, 709).

12. Short coherence interferometer for measuring the partial distances of the eye according to one of the preceding claims, **characterized in that** a so-called rapid scan optical delay line or another path length modulator is also used with the interferometer in the reference or measurement arm.

13. Short coherence interferometer for measuring the partial distances of the eye according to one of the preceding claims, **characterized in that** the initial coincidence of measurement focus and coherence window is placed approximately in the middle of the anterior chamber of the eye or at any other desired location by additional means for adjusting an optical element in the beam path, for example by means of a mirror (306, 747).

14. Short coherence interferometer for measuring the partial distances of the eye according to one of the preceding claims, **characterized in that** the scan stroke is reduced by optical folding of the reference and/or measurement beam paths.

15. Short coherence interferometer for measuring the partial distances of the eye according to one of the preceding claims, **characterized in that** a dispersion compensation is automatically performed **in that** wedge plates arranged in the reference beam path parallel to the movement direction are traversed, and the compensation effect thereby depends on the displacement position.

16. Short coherence interferometer for measuring the partial distances of the eye according to Claim 1, **characterized in that** means are provided for adjusting or orientating the measurement beam axis relative to the optical axis or the visual axis of the eye.

17. Short coherence interferometer for measuring the partial distances of the eye according to one of the preceding claims, **characterized in that** a construction based on the dual beam method is used.

18. Short coherence interferometer for measuring the partial distances of the eye according to one of the preceding claims, **characterized in that** a right-angled mirror or right-angled prism is provided as deflecting element.

**Revendications**

1. Interféromètre à faible longueur de cohérence destiné à mesurer des longueurs de l'oeil, qui focalise le faisceau de mesure sur la fenêtre de cohérence associée respective et/ou qui réduit les distances de balayage nécessaires des miroirs interférométriques à de plus faibles distances que les distances à mesurer, dans lequel il est prévu dans l'interféromètre à faible longueur de cohérence au moins un élément de déviation (par exemple un miroir, un prisme) ayant un angle de déviation α (316, 749) et des éléments d'une optique de focalisation (319, 709) qui effectuent un mouvement périodique de va-et-vient (double flèche 560) de manière à ce que le foyer du faisceau de mesure (101', 322, 712) généré par l'optique de focalisation et dont l'image est formée sur l'oeil au moyen d'une optique relais (103, 321, 711) soit déplacé en synchronisme avec la fenêtre de cohérence de la cornée le long de l'axe optique de l'oeil jusqu'à la rétine (par exemple la fovéa centrale) et retour, **caractérisé en ce qu'**au moins un élément de déviation oriente séquentiellement, lors de son mouvement, le faisceau de mesure ou de référence sur une série de réflecteurs décalés en profondeur et/ou latéralement.

2. Interféromètre à faible longueur de cohérence destiné à mesurer les longueurs de l'oeil selon la revendication 1, **caractérisé en ce que** les réflecteurs décalés en profondeur et/ou latéralement ont une position réglable et/ou **en ce que** le positionnement s'effectue de manière adaptative conformément à des positions nominales préalablement obtenues des surfaces de séparation de l'oeil.

3. Interféromètre à faible longueur de cohérence destiné à mesurer les longueurs de l'oeil selon l'une quelconque des revendications 1 et 2, **caractérisé en ce qu'**au moins un élément de déviation (316, 749) et l'optique de focalisation (319, 709, 709') sont montés l'un derrière l'autre et/ou côte à côte dans la direction de mouvement sur une table effectuant un mouvement périodique de va-et-vient d'un dispositif de balayage.

4. Interféromètre à faible longueur de cohérence destiné à mesurer les longueurs de l'oeil selon l'une quelconque des revendications 1 à 2, **caractérisé en ce qu'**au moins un élément de déviation (316, 749) et l'optique de focalisation (319, 709, 709') sont montés l'un derrière l'autre et/ou côte à côte en formant un angle quelconque par rapport à la direction de mouvement sur la table effectuant un mouvement périodique de va-et-vient d'un dispositif de balayage.

5. Interféromètre à faible longueur de cohérence, destiné à mesurer les longueurs de l'oeil selon l'une quelconque des revendications précédentes, **caractérisé en ce que** l'image du foyer du faisceau de mesure (101, 320, 710, 710') généré par l'optique de focalisation (319, 709, 709') d'une optique relais (321, 711) est formée sur l'oeil, le vertex cornéen étant situé exactement à une distance proche de $b = f \left( 1 + \dfrac{f}{L - D} \right)$ de l'optique relais, avec

   f = distance focale de l'optique relais, L = longueur optique de l'oeil, D = distance des réflecteurs faisant partie de la cornée (317, 617, 750) et de la fovéa centrale (517, 619, 752).

6. Interféromètre à faible longueur de cohérence destiné à mesurer les longueurs de l'oeil selon l'une quelconque des revendications précédentes, **caractérisé en ce que** le foyer du faisceau de mesure (320, 710, 710') généré par l'optique de focalisation (319, 709, 709') est animé d'un mouvement périodique de va-et-vient d'une distance légèrement supérieure à L - D (L = longueur optique de l'oeil ; D = distance des réflecteurs faisant partie de la cornée et de la fovéa centrale).

7. Interféromètre à faible longueur de cohérence destiné à mesurer les longueurs de l'oeil selon l'une quelconque des revendications précédentes, **caractérisé en ce que** la séparation s'effectue dans les bras de mesure et de référence interférométriques (701, 702) de l'interféromètre à faible longueur de cohérence au moyen d'un ou plusieurs coupleurs à fibre optique (700).

8. Interféromètre à faible longueur de cohérence destiné à mesurer les longueurs de l'oeil selon la revendication 1, **caractérisé en ce qu'**au moins un élément de déviation (316, 749) et des éléments de l'optique de focalisation (319, 709, 709') sont montés côte à côte sur des dispositifs de balayage animés de mouvements périodiques de va-et-vient distincts dans la direction de déplacement ou en formant un certain angle par rapport à celle-ci.

9. Interféromètre à faible longueur de cohérence destiné à mesurer les longueurs de l'oeil selon la revendication 1, **caractérisé en ce qu'**au moins un élément de déviation (316, 749) et des éléments de l'optique de focalisation (319, 709, 709') sont montés sur des dispositifs de balayage animés de mouvements périodiques de va-et-vient distincts et **en ce que** les mouvements des deux dispositifs de balayage sont synchronisés de manière électronique, ou **en ce que** les mouvements peuvent être modifiés fonctionnellement l'un par rapport à l'autre.

10. Interféromètre à faible longueur de cohérence destiné à mesurer les longueurs de l'oeil selon l'une quelconque des revendications précédentes, **caractérisé en ce que** le dispositif de balayage (355 avec 356, 725 avec 726, ainsi que 925 et 925' avec 926 et 926') est de préférence une table commandée par moteur pas-à-pas ou moteur piézoélectrique, un dispositif de balayage à bobine vocale ou une table de balayage piézoélectrique à ultrasons.

11. Interféromètre à faible longueur de cohérence destiné à mesurer les longueurs de l'oeil selon l'une quelconque des revendications précédentes, **caractérisé en ce qu'**on utilise en tant qu'optique de focalisation (319, 709) une lentille convergente, une lentille divergente (709') ou un système optique constitué d'une pluralité d'éléments fixes ou variables.

12. Interféromètre à faible longueur de cohérence destiné à mesurer les longueurs de l'oeil selon l'une quelconque des revendications précédentes, **caractérisé en ce qu'**on utilise également avec l'interféromètre dans le bras de référence ou de mesure une ligne dite "ligne à retard optique à balayage rapide" ou un autre modulateur de longueur de trajet.

13. Interféromètre à faible longueur de cohérence destiné à mesurer les longueurs de l'oeil selon l'une quelconque des revendications précédentes, **caractérisé en ce que** la coïncidence initiale du foyer de mesure et de la fenêtre de cohérence est définie approximativement au milieu de la chambre antérieure de l'oeil ou à un autre emplacement quelconque à l'aide de moyens supplémentaires destinés à l'ajustement d'un élément optique dans le chemin de faisceau, par exemple au moyen d'un miroir (306, 747).

14. Interféromètre à faible longueur de cohérence destiné à mesurer les longueurs de l'oeil selon l'une quelconque des revendications précédentes, **caractérisé en ce que** la course de balayage est réduite par repliement du chemin du faisceau de référence et/ou de mesure.

15. Interféromètre à faible longueur de cohérence destiné à mesurer les longueurs de l'oeil selon l'une quelconque des revendications précédentes, **caractérisé en ce qu'**une compensation automatique de la dispersion est effectuée en plaçant sur le chemin du faisceau de référence des plaques de calage disposées parallèlement à la direction de mouvement de manière à ce que l'effet de compensation dépende de la position de décalage.

16. Interféromètre à faible longueur de cohérence destiné à mesurer les longueurs de l'oeil selon la revendication 1, **caractérisé en ce qu'**il est prévu des moyens destinés à incliner ou à orienter l'axe du faisceau de mesure par rapport à l'axe optique ou à l'axe de vision de l'oeil.

17. Interféromètre à faible longueur de cohérence destiné à mesurer les longueurs de l'oeil selon l'une quelconque des revendications précédentes, **caractérisé en ce qu'**on utilise une structure conforme au procédé du double faisceau.

18. Interféromètre à faible longueur de cohérence destiné à mesurer les longueurs de l'oeil selon l'une quelconque des revendications précédentes, **caractérisé en ce qu'**il est prévu en tant qu'élément de déviation un miroir ou un prisme à 90°.

Figur 1

Figur 2

Figur 3

Figur 4

EP 1 713 378 B1

Figur 5

Figur 6

Figur 7

Figur 8

EP 1 713 378 B1

Figur 9

Figur 10

Figur 11

Figur 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3201801 A1
- WO 0138820 A1
- DE 19624167 A1
- WO 02065899 A2

**In der Beschreibung aufgeführte Nicht-Patentliteratur**

- **K. F. KWONG et al.** 400-Hz mechanical scanning optical delay line. *Optics Letters,* 1993, vol. 18, 558-560